# EUROPEAN PATENT SPECIFICATION

(11) **EP 3 151 761 B1**
(45) Date of publication and mention of the grant of the patent: **04.12.2019**
(21) Application number: 15729681.5
(22) Date of filing: 02.06.2015
(51) Int. Cl.: A61B 17/16

(54) **ABRASION TOOL WITH VACUUM SOURCE**
SCHLEIFWERKZEUG MIT VAKUUMQUELLE
OUTIL D'ABRASION AVEC SOURCE DE VIDE

(30) Priority: 04.06.2014 US 201462007674 P
(43) Date of publication of application: 12.04.2017
(73) Proprietor: Smith & Nephew, Inc, Memphis, TN 38116 (US)
(72) Inventor: BOILEAU, Pascal, 06200 Nice (FR); STREIT, Oliver, 78532 Tuttlingen (DE)
(74) Representative: Smith & Nephew
(86) International application number: PCT/US2015/033704
(87) International publication number: WO 2015/187643

(56) References cited:
- EP-A1- 0 958 787
- US-A- 1 983 601
- US-A1- 2001 037 114
- US-A1- 2006 129 159

## Description

### BACKGROUND

Bones are composed of a hard, outer layer of relatively dense bone tissue, referred to as cortical bone, and a relatively soft bone including blood vessels and marrow, referred to as spongy or cancellous bone. When performing surgical operations to repair bone, for example, repairing bone loss by a bone graft, the cancellous bone is exposed and abraded over the surface to be repaired. The abrasion of the cancellous bone helps stimulate bone growth within the abraded region.

Typically, a rasp, burr, or other tool including a hard, rough surface is employed to abrade the bone. However, existing tools generate bubbles in body fluids, causing debris removed from the bone to accumulate. As a result, it becomes difficult to maintain adequate visibility within the region of the abraded bone. Thus, it is desirable to provide tools for abrading bone surfaces that provide the ability to remove debris cast off from the bone during abrasion.

US1983601 describes a mucus surface scraper and medicinal distributor for use either as a scraping cleanser or a distributor of antiseptic solutions. It is directed to the problem of cleansing surfaces of the crevices and fissures between papillae of the tongue.

EP0958787 describes a tongue scraper adapted to suck a tongue surface to a cylindrical member by a suction system while scraping the tongue surface with spatulate members and/or brushes. Papillae on the tongue surface are successively raised or laid down by scraping the tongue surface with the spatulate member and brushes.

US2006129159 describes a facial bone contouring device for use in facial bone contouring surgery and bone tumor and/or osteophyte removal. The facial bone contouring device comprises: a rasp having a double tube structure, including a rod, and a cutter provided with plural grooves for exhausting cut bone fragments.

US2001037114 describes a handpiece comprising a cutting member which itself includes a surgical suction rasp for being reciprocatively driven to cut anatomical tissue and having a tissue cutting surface and a suction passage with an inlet opening along the tissue cutting surface for removing anatomical debris from an operative site at which the rasp is used.

### Summary

Abrasion tools have been developed that include suction mechanisms for removal of debris from the vicinity of the tool. However, these devices are also problematic. For example, existing tools do not extract body fluids and other debris directly at the location where bone abrasion occurs. Thus, visibility within the region of abraded bone remains limited. Furthermore, existing abrasion tools possess rounded abrasion surfaces and do not provide a flat bone surface for repair. The absence of a flat surface on the bone to be repaired limits contact between the prepared bone surface and a bone graft. Accordingly, there exists a continued need for bone abrading tools that provide improved debris removal from an abrasion site and attendant visibility for surgeons.

According to a first aspect of the invention, a surgical rasp tool for abrading bone as defined by claim 1 is provided. It includes a generally elongate body having a proximal end and a distal end, the proximal end including a first proximal segment, a head formed in the distal end of the body, and a lumen formed within the body and extending from the first proximal segment to the head. The head further includes an opening spanning a portion of the width of the head and in communication with the lumen and a plurality of teeth extending transversely to the longitudinal axis and spanning the width of the opening, where the plurality of teeth are longitudinally spaced apart by a plurality of gaps. The first proximal segment of the body is further adapted to communicate with a vacuum source and wherein the lumen is adapted to convey a vacuum force applied by the vacuum source from the first proximal segment to the plurality of gaps.

Further embodiments of the tool may include one or more of the following, alone or in combination. The tool body may include a bend positioned proximal to the head, where a longitudinal axis of the body is not aligned with the longitudinal axis of the tool body. The tool body may further include a second proximal segment, where the second proximal segment and the distal end of the body extend along a longitudinal axis of the body. The second proximal segment may be adapted to engage with a handle for manipulation of the tool. The first proximal segment may be oriented transversely with respect to the longitudinal axis of the body. The first proximal segment may be further formed with or adapted to receive a Luer lock or any kind of adaptor for mating with a corresponding Luer lock or any kind of adaptor in communication with the vacuum source. The lumen may be accessible only from the plurality opening in the head and the first proximal segment. The lumen may be integrally formed within the body. The teeth may be adapted to inhibit permanent deformation or fracture when in contact with bone. The plurality of gaps may possess a width selected within the range between about 0.2 mm to about 3 mm.

According to a second aspect of the invention, a bone abrasion tool as defined by claim 8 is provided. It includes a generally elongate body having a proximal end and a distal end, a head formed at the distal end of the body, and a channel formed within the body, the channel extending proximally from the head and transversely through a first surface of the body. The head further includes an opening formed through a second surface of the body, opposite the first body surface, and in communication with the channel, the opening spanning a portion of the width of the head, and a plurality of teeth extending transversely to the longitudinal axis and spanning the width of the opening, where the plurality of teeth are longitudinally spaced apart by a plurality of gaps. The tool may further include a tube dimensioned for receipt within the channel, the tube extending between a proximal end and a distal end, where the distal end of the tube includes an opening adapted to communicate with the opening in the distal end of the body when the tube is inserted within the channel and where the proximal end of the tube is further adapted to communicate with a vacuum source. The tube may be further adapted to convey a vacuum force applied by the vacuum source from the proximal end of the tube to the plurality of gaps when the tube is inserted within the channel.

Further embodiments of the tool may include one or more of the following, alone or in combination. The tool body may further include a bend positioned proximal to the head, where an longitudinal axis of the body is not aligned with the longitudinal axis of the tool body. The proximal end of the body and the distal end of the body may extend along a longitudinal axis of the body. The proximal end of the body may be adapted to engage with a handle for manipulation of the tool. The channel may increase in depth towards the head. The proximal end of the tube may be further oriented in a direction transverse to the longitudinal axis of the body when the tube is inserted within the channel. The proximal end of the tube may be formed with, or adapted to receive, a Luer lock for mating with a corresponding Luer lock in communication with the vacuum source. The tube may be substantially fluid tight and accessible only from the proximal end of the tube and the distal end of the tube. The teeth may be dimensioned so as to inhibit deformation or fracture when in contact with bone. The plurality of gaps may possess a width selected within the range between about 0.2 mm to about 3 mm.

### BRIEF DESCRIPTION OF THE DRAWINGS

The foregoing and other objects, features and advantages will be apparent from the following more particular description of the embodiments, as illustrated in the accompanying drawings in which like reference characters refer to the same parts throughout the different views. The drawings are not necessarily to scale, emphasis instead being placed upon illustrating the principles of the embodiments.
Figures 1A-1B are 3-D solid renderings of an embodiment of an abrasion tool of the present disclosure having an integrally formed suction channel;
Figures 2A-2C are 3-D solid renderings of another embodiment of an abrasion tool of the present disclosure;
Figures 3A-3C are schematic illustrations of a tube of the tool of Figures 2A-2C in isolation and showing example dimensions;
Figures 4A-4C are schematic illustrations of a body of the tool of Figures 2A-2C in isolation and showing example dimensions;
Figures 5A-5B are schematic illustrations of a fluid lock of the tool of Figures 2A-2C in isolation and showing example dimensions;
Figure 6 is a photograph illustrating an embodiment of the tool of the present disclosure including teeth of varying width; and
Figures 7A-7B are schematic illustrations of alternative embodiments of the tool body.

### DETAILED DESCRIPTION

Examples of the abrasion tool will now be discussed with reference to the figures.

In the description that follows, like components have been given the same reference numerals, regardless of whether they are shown in different examples. To illustrate example(s) in a clear and concise manner, the drawings may not necessarily be to scale and certain features may be shown in somewhat schematic form. Features that are described and/or illustrated with respect to one example may be used in the same way or in a similar way in one or more other examples and/or in combination with or instead of the features of the other examples.

Comprise, include, and/or plural forms of each are open ended and include the listed parts and can include additional parts that are not listed. And/or is open ended and includes one or more of the listed parts and combinations of the listed parts.

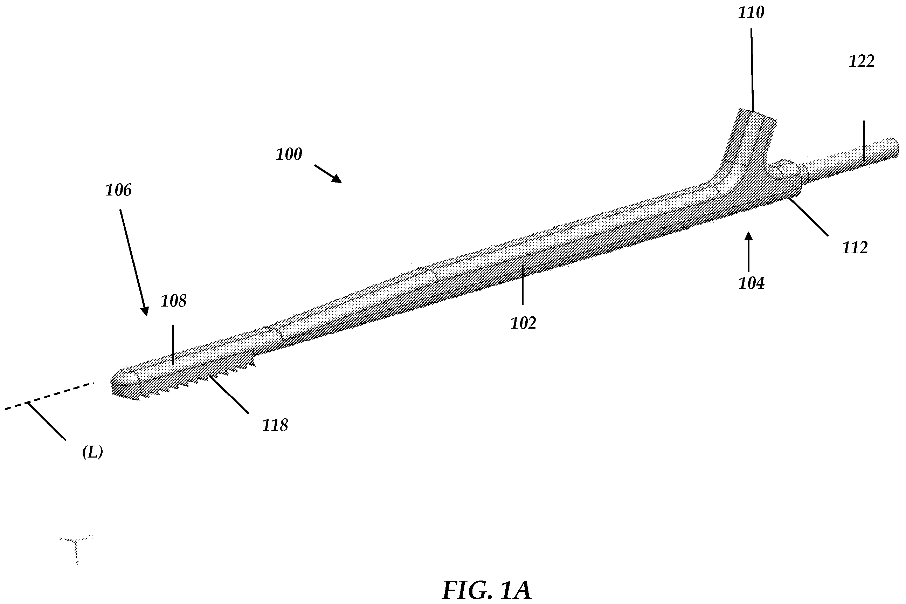

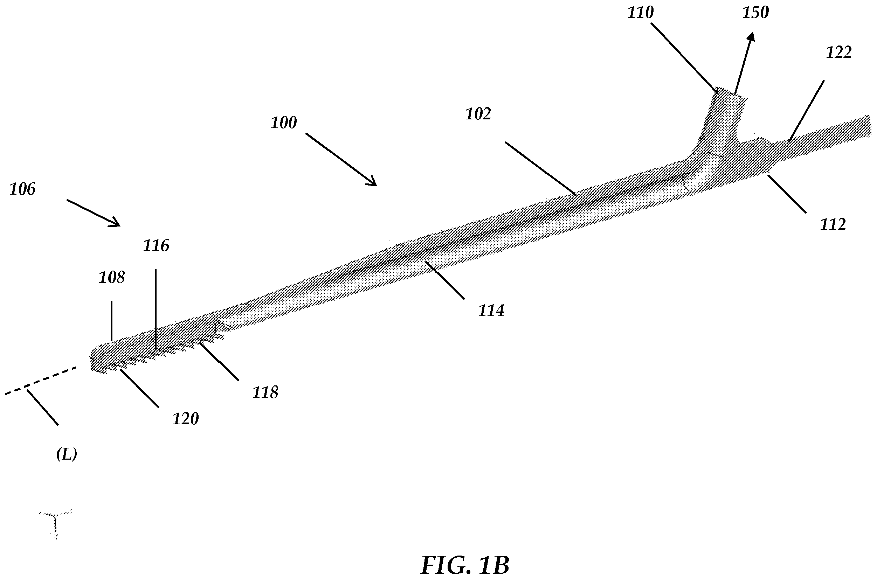

The discussion will turn to Figure 1, which illustrates a solid renderings of a first embodiment of an abrasion tool 100 of the present disclosure, also referred to as a rasp. The tool 100 includes a generally elongate body 102 having a proximal end 104 and a distal end 106, the proximal end 104 including a first proximal segment 110 and second proximal segment 112, as discussed in greater detail below. The tool 100 further includes a head 108 formed in the distal end 106 of the body 102. The first proximal segment 110 is oriented transversely with respect to the longitudinal axis (L) of the body 102. This deflection advantageously allows clearance for a placement of a locking mechanism, allowing connection of a lumen with a vacuum source, as discussed in greater detail below.

As shown in Figure 1B, a lumen 114 is further formed within the body 102 and extends from the first proximal segment 110 to the head 108. As discussed in greater detail below, the first proximal segment 110 is adapted to communicate with a vacuum source 150 and the lumen 114 allows transmission of a vacuum force generated by the vacuum source 150 along the length of the body 102, from the head 108 to the first proximal segment 110. The longitudinal axis (L) of the body 102 extends between the distal end 106 of the body 102 and the second proximal segment 112. The second proximal segment 112 is further adapted to engage with a handle (not shown), allowing a user to manipulate the tool 100 by grasping and moving the handle. In certain embodiments, the second proximal segment 112 may engage one end of an adapter 122 connected to the handle. In other embodiments, the handle may engage the second proximal segment 112 directly. The head 108 includes an opening 116 spanning a portion of the width of the head 108. The opening 116 faces outward from the body 102 in a direction transverse to the longitudinal axis (L). The opening 116 is further in communication with a distal end of the lumen 114.

A plurality of teeth 118 are positioned within the head 108, spanning the opening 116 and extending outward from the opening 116, also transverse to the longitudinal axis (L) of the body 102. The plurality of teeth 118 are separated from one another by a plurality of gaps 120. The gaps 120 advantageously allow communication between the region outside the tool 100 with the opening 116 and the lumen 114. The gaps 120 are further dimensioned so as to allow the passage, via the vacuum, of objects (e.g., fluid, solid debris removed from a bone surface). For example, in certain embodiments, the gaps 120 may possess a width, in the direction of the longitudinal axis (L) of the body 102, selected within the range between about 0.2 mm to about 3.0 mm. However, it may be understood that, in alternative embodiments, the dimensions of the gaps 120 may be provided within alternative ranges, as necessary. In certain embodiments, the lumen 114 is integrally formed within the body 102. Thus, the lumen 114 is accessible only from the opening 116 and the first proximal segment 110. Furthermore, the lumen 114 is generally between the opening 116 and the first proximal segment 110 (i.e., along the length of the body 102).

The first proximal segment 110 of the body 102 is further adapted to communicate with the vacuum source 150. For example, the first proximal segment 110 may be formed with, or adapted to receive, a locking mechanism such as a Luer lock (e.g., a female Luer lock) or other adaptor. So configured, a proximal end of the locking mechanism may engage a mating locking mechanism such as a Luer lock (e.g., a male Luer lock) or other adaptor for communication with the vacuum source 150. A vacuum force applied by the vacuum source 150 in communication with the first proximal segment 110 may be transmitted along the lumen 114 from the first proximal segment 110 to the plurality of gaps 120 in the teeth 118. Thus, debris adjacent the plurality of teeth 118 may be urged into the lumen 114 through the gaps 120 and through the lumen 114 under the influence of the vacuum force, removing the debris from the region surrounding the teeth 118 and advantageously providing improved visibility of the head 108 during surgery.

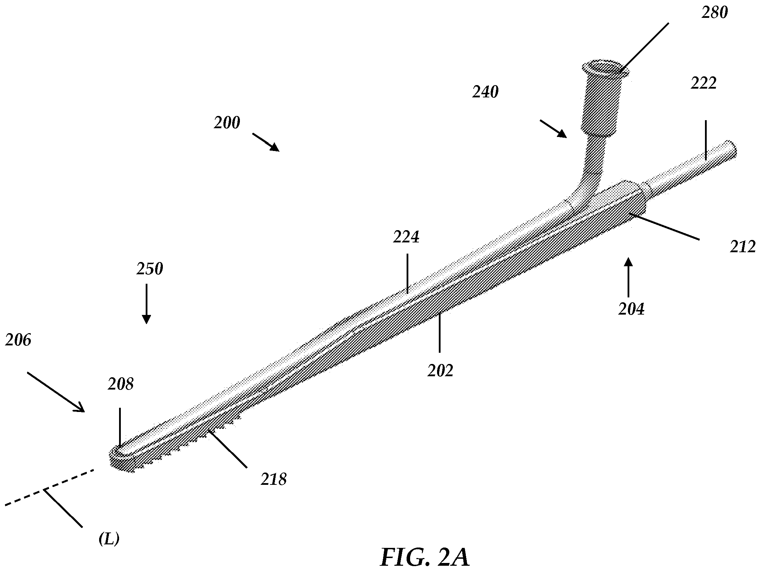

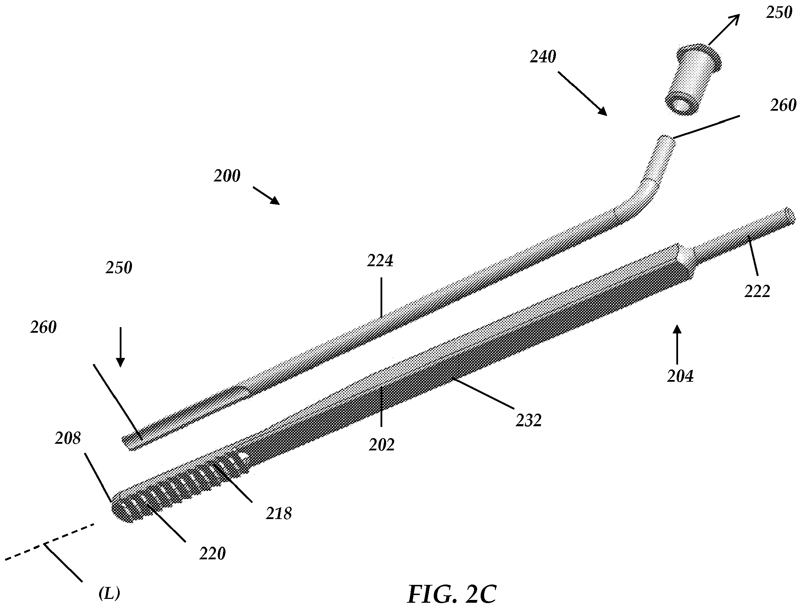

The discussion will turn to Figures 2A-2C, which illustrate solid renderings of a second embodiment of the abrasion tool 200 of the present disclosure. The tool 200 includes a generally elongate body 202 having a proximal end 204 and a distal end 206. The tool 200 further includes a tube 224, separately formed from the body 202 and adapted to mate therewith. As discussed below, the tube 224 serves the role of the lumen 114 of the first tool 100 embodiment of Figures 1A-1B, allowing transmission of a vacuum force from a proximal end 240 of the tube 224 to the head 208 and removal of debris through the tube 224 under the influence of the vacuum force.

The longitudinal axis (L) of the body 202 extends between the distal end 206 of the body 202 and the second proximal segment 212. The second proximal segment 212 is further adapted to engage with a handle (not shown), allowing a user to manipulate the tool 200 by grasping and moving the handle. In certain embodiments, the second proximal segment 212 may engage one end of an adapter 222 connected to the handle. In other embodiments, the handle may engage the second proximal segment 212 directly. A channel 226 is formed within the body 202 and extends proximally from the head 208. The channel 226 further extends through a first surface 230 of the body 202, allowing the tube 224 to be seated therein. In certain embodiments, the channel 226 is tapered, increasing in depth towards the distal end 206 of the body 202.

The tool 200 further includes a head 208 formed in the distal end 206 of the body 202. The head 208 includes an opening 216 spanning a portion of the width of the head 208. The opening 216 faces outward from the tool body 202 through a second surface 232 of the body 202. The second surface 232 opposes the first surface 230 and faces a direction transverse to the longitudinal axis (L). The tube 224 possesses tube openings 260 at each end. Accordingly, the tube 224 is fully cannulated. In certain embodiments, the tube 224 may be accessed only from the tube openings 260 and is fluid tight, allowing fluid passage between its proximal end 240 and distal end 250.

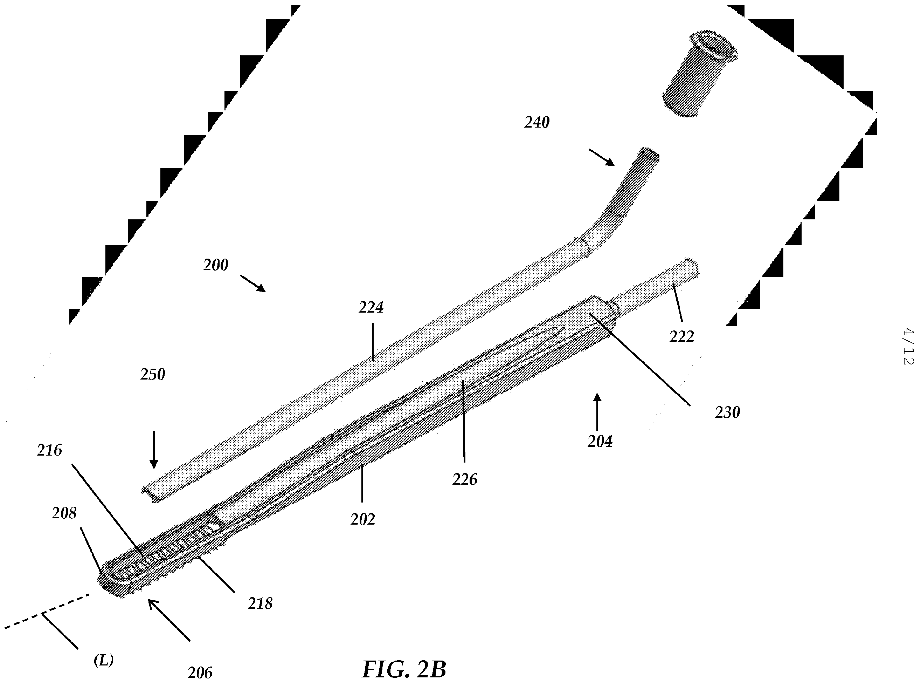

At least a portion of the tube 224 is further adapted to be received within the channel 226 of the body 202 (Figure 2B). For example, the tube 224 may extend approximately straight from the distal end 240 along a portion of its length. The tube 224 may be further formed with one or more bends. A first bend may be formed near the proximal end 240 of the tube 224. The first bend advantageously provides clearance for a placement of a locking mechanism at the proximal end 240 of the tube 224, allowing connection of the tube 224 with a vacuum source, as discussed in greater detail below. Thus, when the distal end 250 of the tube 224 is positioned within the channel 226, the proximal end 240 of the tube 224 is oriented in a direction transverse to the longitudinal axis (L) of the body 202 and does not lie within the channel 226. As illustrated in the embodiment of Figure 2A, when the tube 224 is seated within the body 202, the opening 260 in the distal end 250 of the tube 224 overlies the opening 216, allowing communication of a vacuum force from the opening 260 in the distal end 250 of the tube 224 to the opening 216.

A plurality of teeth 218 are positioned within the head 208, spanning the opening 216 and extending outward from the opening 216, also transverse to the longitudinal axis (L) of the body 202. The plurality of teeth 218 are separated from one another by a plurality of gaps 220. The gaps 220 allow communication between a region outside the tool 200 with the opening 216 and the opening 260 in the distal end 250 of the tube 224 when the tube 224 is seated within the channel 226. The gaps 220 are further dimensioned so as to allow the passage of objects (e.g., fluid, solid debris removed from a bone surface). For example, the gaps 220 may possess a width, in the direction of the longitudinal axis (L) of the body 202, within the range between about 0.2 mm to about 3.0 mm. However, it may be understood that, in alternative embodiments, the dimensions of the gaps 220 may be provided within alternative ranges, as necessary.

In embodiments of the second tool 200, the proximal end 240 of the tube 224 is further adapted to communicate with a vacuum source, allowing transmission of a vacuum force generated by the vacuum source along the length of the tube 224. For example, the proximal end 240 of the tube 224 may be formed with, or adapted to receive, a locking mechanism 280 such as a Luer lock (e.g., a female Luer lock) or other adaptor. So configured, a proximal end of the locking mechanism may engage a mating locking mechanism such as a Luer lock (e.g., a male Luer lock) or other adaptor for communication with the vacuum source 250. A vacuum force applied by the vacuum source 250 may be transmitted along the tube 224 from tube opening 260 in the proximal end 240 to the tube opening 260 in the distal end 250 to the gaps 220 between the teeth 218. Thus, debris adjacent the plurality of teeth 218 may be urged through the gaps 220 and through the tube 224 under the influence of the vacuum force, removing the debris from the region surrounding the teeth 218 and advantageously providing improved visibility of the head 208 during surgery.

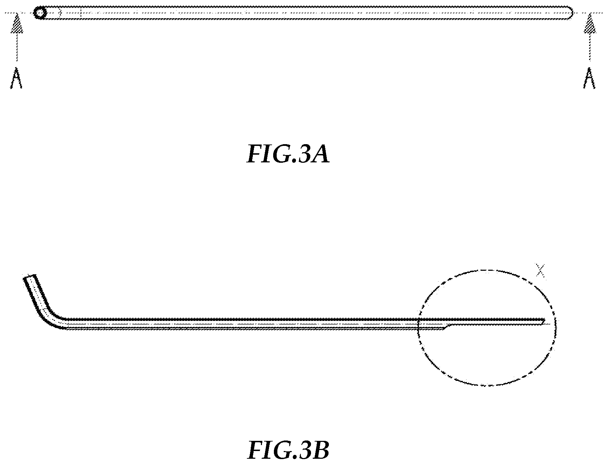

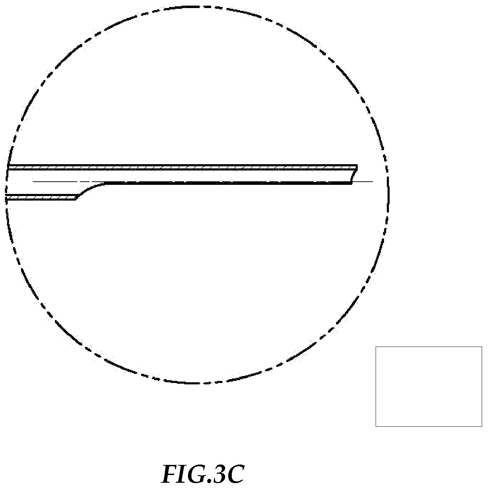

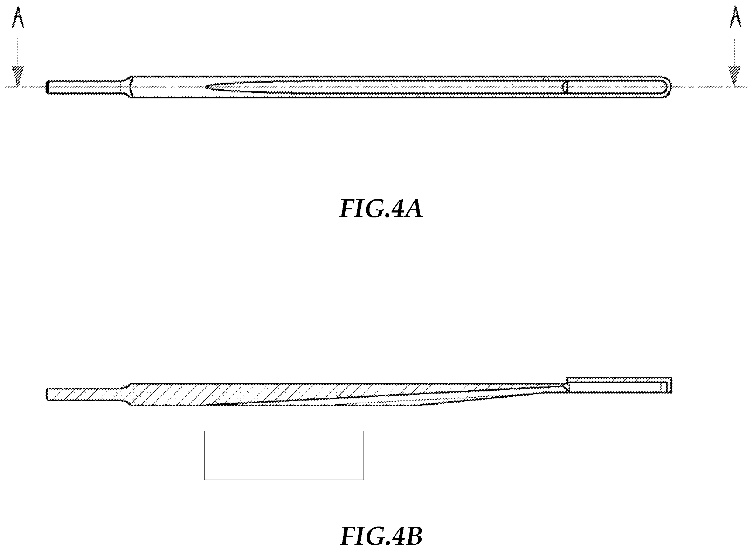

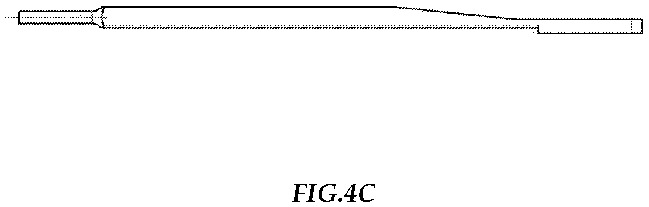

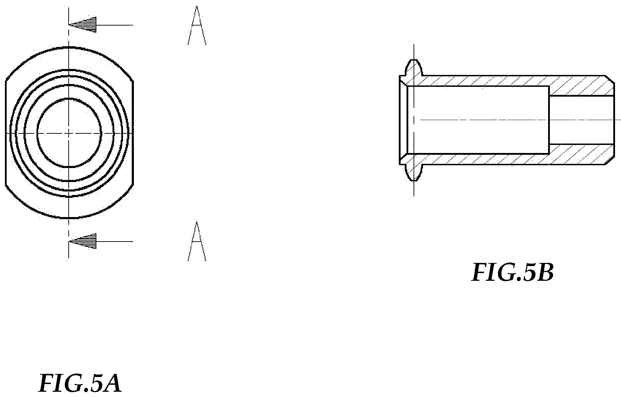

Figures 3A-3C, 4A-4C, and 5A-5B present further schematic illustrations of the tool of Figures 2A-2C and provide dimensions of the tool in certain embodiments. It may be understood, however, that these dimensions are provided for example purposes and that the tool may adopt other dimensions, as necessary.

With regard to both of the tool embodiments of Figures 1A-1C and 2A-2C, the teeth 118, 218 may be further adapted to inhibit permanent deformation and/or fracture when in contact with bone. In an embodiment, the teeth 118, 218 may be formed such that they remain within the elastic regime under the stress experienced when contacting bone (e.g., shear stresses, tensile stresses, compressive stresses). This may be accomplished by the selection of material forming the teeth 118, 218, the dimensions of the teeth 118, 218, etc.

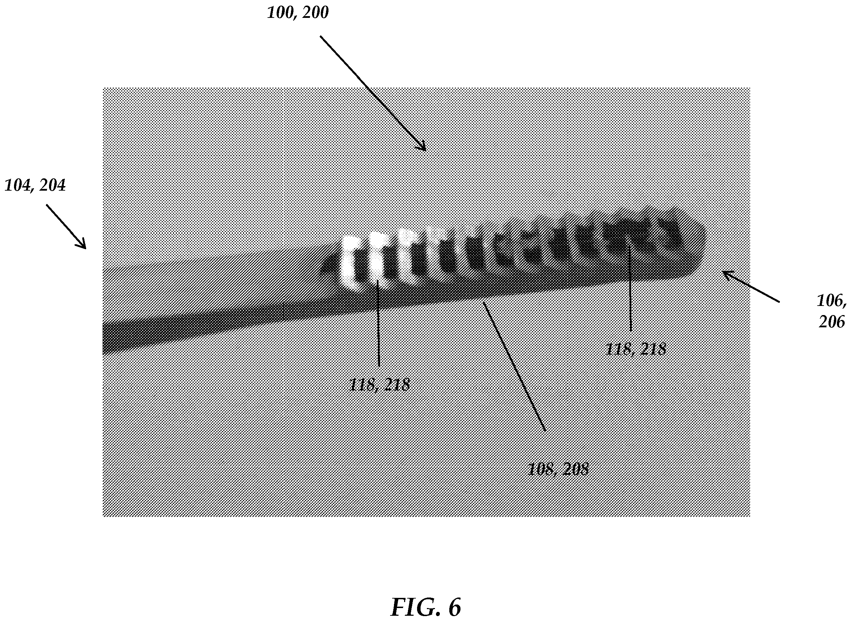

For example, with reference to Figure 6, a photograph of an embodiment of the teeth 118, 218 is illustrated after use abrading a bone. The geometry of the teeth 118, 218 is kept constant along the length of the head 108, 208, except for the width, where the tooth width increase moving proximally. It may be observed that the relatively narrow teeth 118, 218 positioned near the distal end 106, 206 of the tool 100, 200 have fractured, while the wider teeth 118, 218 positioned near the middle of the head 108, 208 and proximal end 104, 204 of the tool 100, 200 remain intact and exhibit no permanent deformation. As a result, it may be understood that a maximum tooth width may be advisable, for a given tooth material, in order to reduce the likelihood of tooth failure or permanent deformation.

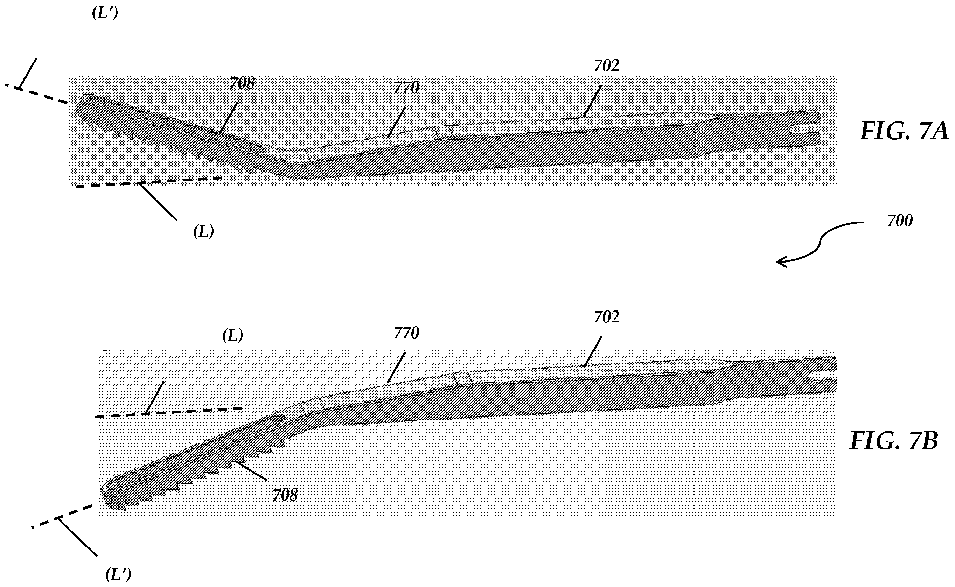

Alternative embodiments of the body 100, 200 are further illustrated in Figures 7A-7B. Figures 7A-7B illustrate a body 700 having a bend 770, such that a longitudinal axis (L') of the head 708 is not aligned (e.g., not parallel) with respect to the longitudinal axis (L) of the body 702. Such configurations may accommodate different patients or surgical procedures. For example, with regards to the embodiment of Figure 7A, the head 708 is angled upwards relative to the body 702 such that a longitudinal axis (L') of the head 708 forms a positive angle with respect to the longitudinal axis (L) of the body 702. In the embodiment of Figure 7B, the head 708 is angled downwards, relative to the body 702, such that the longitudinal axis (L) of the head 708 forms a negative angle with respect to the longitudinal axis (L') of the body 702. Embodiments of the body 702 of Figures 7A-7B may be employed in combination with either of the embodiments illustrated and discussed with regards to Figures 1A-1B and 2A-2C.

The terms comprise, include, and/or plural forms of each are open ended and include the listed parts and can include additional parts that are not listed. The term and/or is open ended and includes one or more of the listed parts and combinations of the listed parts.

One skilled in the art will realize the invention may be embodied in other specific forms. The foregoing embodiments are therefore to be considered in all respects illustrative rather than limiting of the invention described herein. Scope of the invention is thus indicated by the appended claims, rather than by the foregoing description, and all changes that come within the range of the claims are therefore intended to be embraced therein.

## Claims

1. A surgical rasp tool (100) for abrading bone, the tool comprising:
a generally elongate body (102) having a proximal end (104), a distal end (106), and a longitudinal axis, the proximal end (104) including a first proximal segment (110);
a head (108) formed in the distal end of the body (102);
a lumen (114) formed within the body and extending from the first proximal segment (110) to the head;
the head further including:
an opening (116) spanning a portion of the width of the head and in communication with the lumen;
a plurality of teeth (118) extending transversely to the longitudinal axis and spanning the width of the opening, wherein the plurality of teeth are longitudinally spaced apart by a plurality of gaps (120);
wherein the first proximal segment of the body (110) is further adapted to communicate with a vacuum source and wherein the lumen is adapted to convey a vacuum force applied by the vacuum source from the first proximal segment (110) to the plurality of gaps (120).

2. The tool of Claim 1, wherein the body further comprises a bend positioned proximal to the head and wherein a longitudinal axis of the body is not aligned with the longitudinal axis of the head.

3. The tool of Claim 1, wherein the body further includes a second proximal segment, the second proximal segment and the distal end of the body extending along a longitudinal axis of the body, for example wherein the second proximal segment is adapted to engage with an attachment for manipulation of the tool, for example wherein the first proximal segment is oriented transversely with respect to the longitudinal axis of the body, for example wherein the first proximal segment is further formed with or adapted to receive a Luer lock or any kind of adaptor for mating with a corresponding Luer lock or any kind of adaptor in communication with the vacuum source.

4. The tool of any of Claims 1-3, wherein the lumen is accessible only from the plurality of openings in the head and the first proximal segment.

5. The tool of any of Claims 1-4, wherein the lumen is integrally formed within the body.

6. The tool of any of Claims 1-5, wherein the teeth are adapted to inhibit permanent deformation or fracture when in contact with bone.

7. The tool of any of Claims 1-6, wherein the plurality of gaps possess a width selected within the range between about 0.2 mm to about 3 mm.

8. A bone abrasion tool (200), comprising:
a generally elongate body having a proximal end (204), a distal end (206), and a longitudinal axis;
a head (208) formed at the distal end of the body (202);
a channel formed within the body, the channel extending proximally from the head and transversely through a first surface of the body;
the head further including:
an opening formed through a second surface of the body, opposite the first surface, and in communication with the channel, the opening spanning a portion of the width of the head;
a plurality of teeth extending transversely to the longitudinal axis and spanning the width of the opening, wherein the plurality of teeth are longitudinally spaced apart by a plurality of gaps; and
a tube dimensioned for receipt within the channel, the tube extending between a proximal end and a distal end, wherein the distal end of the tube includes an opening adapted to communicate with the opening in the distal end of the body when the tube is inserted within the channel and wherein the proximal end of the tube is further adapted to communicate with a vacuum source;
wherein the tube is further adapted to convey a vacuum force applied by the vacuum source from the proximal end of the tube to the plurality of gaps when the tube is inserted within the channel.

9. The tool of Claim 8, wherein the body further comprises a bend positioned proximal to the head and wherein a longitudinal axis of the body is not aligned with the longitudinal axis of the head.

10. The tool of Claim 8, wherein the proximal end of the body and the distal end of the body extend along a longitudinal axis of the body.

11. The tool of Claim 8, wherein the proximal end of the body is adapted to engage with an attachment for manipulation of the tool, for example wherein the channel increases in depth towards the head and wherein the proximal end of the tube is oriented in a direction transverse to the longitudinal axis of the body when the tube is inserted within the channel.

12. The tool of any of Claims 8-11, wherein the first proximal end of the tube is further formed with or adapted to receive a Luer lock or any kind of adaptor for mating with a corresponding Luer lock or any kind of adaptor in communication with the vacuum source.

13. The tool of any of Claims 8-12, wherein the tube is substantially fluid tight and accessible only from the proximal end of the tube and the distal end of the tube.

14. The tool of any of Claims 8-13, wherein the teeth are dimensioned so as to inhibit deformation or fracture when in contact with bone.

15. The tool of any of Claims 8-14, wherein the plurality of gaps possess a width selected within the range between about 0.2 mm to about 3 mm.

## Patentansprüche

1. Ein chirurgisches Feilenwerkzeug (100) zum Abschleifen von Knochen, wobei das Werkzeug Folgendes beinhaltet:
einen im Allgemeinen länglichen Körper (102) mit einem proximalen Ende (104), einem distalen Ende (106) und einer Längsachse, wobei das proximale Ende (104) ein erstes proximales Segment (110) umfasst;
einen Kopf (108), der in dem distalen Ende des Körpers (102) gebildet ist;
ein Lumen (114), das innerhalb des Körpers gebildet ist und sich von dem ersten proximalen Segment (110) zu dem Kopf erstreckt;
wobei der Kopf ferner Folgendes umfasst:
eine Öffnung (116), die einen Abschnitt der Breite des Kopfes überspannt und mit dem Lumen in Verbindung steht;
eine Vielzahl von Zähnen (118), die sich quer zu der Längsachse erstrecken und die Breite der Öffnung überspannen, wobei die Vielzahl von Zähnen der Länge nach durch eine Vielzahl von Lücken (120) beabstandet ist;
wobei das erste proximale Segment des Körpers (110) ferner angepasst ist, um mit einer Vakuumquelle in Verbindung zu stehen, und wobei das Lumen angepasst ist, um eine von der Vakuumquelle angewendete Vakuumkraft von dem ersten proximalen Segment (110) zu der Vielzahl von Lücken (120) zu übertragen.

2. Werkzeug gemäß Anspruch 1, wobei der Körper ferner eine Biegung beinhaltet, die proximal zu dem Kopf positioniert ist, und wobei eine Längsachse des Körpers nicht nach der Längsachse des Kopfes ausgerichtet ist.

3. Werkzeug gemäß Anspruch 1, wobei der Körper ferner ein zweites proximales Segment umfasst, wobei sich das zweite proximale Segment und das distale Ende des Körpers entlang einer Längsachse des Körpers erstrecken, wobei zum Beispiel das zweite proximale Segment angepasst ist, um in ein Zubehörteil zur Bedienung des Werkzeugs einzugreifen, wobei zum Beispiel das erste proximale Segment in Bezug auf die Längsachse des Körpers quer orientiert ist, wobei zum Beispiel das erste proximale Segment ferner mit Folgendem gebildet ist oder angepasst ist, um Folgendes aufzunehmen: ein Luer-Lock oder eine beliebige Art von Adapter zum Zusammenpassen mit einem entsprechenden Luer-Lock oder einer beliebigen Art von Adapter in Verbindung mit der Vakuumquelle.

4. Werkzeug gemäß einem der Ansprüche 1-3, wobei das Lumen nur von der Vielzahl von Öffnungen in dem Kopf und dem ersten proximalen Segment zugänglich ist.

5. Werkzeug gemäß einem der Ansprüche 1-4, wobei das Lumen innerhalb des Körpers einstückig gebildet ist.

6. Werkzeug gemäß einem der Ansprüche 1-5, wobei die Zähne angepasst sind, um eine dauerhafte Verformung oder einen Bruch zu verhindern, wenn sie mit Knochen in Kontakt kommen.

7. Werkzeug gemäß einem der Ansprüche 1-6, wobei die Vielzahl von Lücken eine Breite besitzen, die innerhalb des Bereichs zwischen etwa 0,2 mm bis etwa 3 mm ausgewählt ist.

8. Ein Knochenabschleifwerkzeug (200), beinhaltend:
einen im Allgemeinen länglichen Körper mit einem proximalen Ende (204), einem distalen Ende (206) und einer Längsachse;
einen Kopf (208), der an dem distalen Ende des Körpers (202) gebildet ist;
einen Kanal, der innerhalb des Körpers gebildet ist, wobei sich der Kanal proximal von dem Kopf und quer durch eine erste Oberfläche des Körpers erstreckt;
wobei der Kopf ferner Folgendes umfasst:
eine Öffnung, die durch eine zweite Oberfläche des Körpers gebildet ist, gegenüberliegend der ersten Oberfläche, und mit dem Kanal in Verbindung steht, wobei die Öffnung einen Abschnitt der Breite des Kopfes überspannt;
eine Vielzahl von Zähnen, die sich quer zu der Längsachse erstrecken und die Breite der Öffnung überspannen, wobei die Vielzahl von Zähnen der Länge nach durch eine Vielzahl von Lücken beabstandet ist; und
ein Röhrchen, das zur Aufnahme innerhalb des Kanals bemessen ist, wobei sich das Röhrchen zwischen einem proximalen Ende und einem distalen Ende erstreckt, wobei das distale Ende des Röhrchens eine Öffnung umfasst, die zur Verbindung mit der Öffnung in dem distalen Ende des Körpers angepasst ist, wenn das Röhrchen innerhalb des Kanals eingefügt ist, und wobei das proximale Ende des Röhrchens ferner angepasst ist, um mit einer Vakuumquelle in Verbindung zu stehen;
wobei das Röhrchen ferner angepasst ist, um eine von der Vakuumquelle angewendete Vakuumkraft von dem proximalen Ende des Röhrchens zu der Vielzahl von Lücken zu übertragen, wenn das Röhrchen innerhalb des Kanals eingefügt ist.

9. Werkzeug gemäß Anspruch 8, wobei der Körper ferner eine Biegung beinhaltet, die proximal zu dem Kopf positioniert ist, und wobei eine Längsachse des Körpers nicht nach der Längsachse des Kopfes ausgerichtet ist.

10. Werkzeug gemäß Anspruch 8, wobei sich das proximale Ende des Körpers und das distale Ende des Körpers entlang einer Längsachse des Körpers erstrecken.

11. Werkzeug gemäß Anspruch 8, wobei das proximale Ende des Körpers angepasst ist, um in ein Zubehörteil zur Bedienung des Werkzeugs einzugreifen, wobei zum Beispiel die Tiefe des Kanals zu dem Kopf hin zunimmt und wobei das proximale Ende des Röhrchens in einer Richtung quer zu der Längsachse des Körpers orientiert ist, wenn das Röhrchen innerhalb des Kanals eingefügt ist.

12. Werkzeug gemäß einem der Ansprüche 8-11, wobei das erste proximale Ende des Röhrchens ferner mit Folgendem gebildet ist oder angepasst ist, um Folgendes aufzunehmen: ein Luer-Lock oder eine beliebige Art von Adapter zum Zusammenpassen mit einem entsprechenden Luer-Lock oder einer beliebigen Art von Adapter in Verbindung mit der Vakuumquelle.

13. Werkzeug gemäß einem der Ansprüche 8-12, wobei das Röhrchen im Wesentlichen fluiddicht ist und nur von dem proximalen Ende des Röhrchens und dem distalen Ende des Röhrchens zugänglich ist.

14. Werkzeug gemäß einem der Ansprüche 8-13, wobei die Zähne bemessen sind, um eine Verformung oder einen Bruch zu verhindern, wenn sie mit Knochen in Kontakt kommen.

15. Werkzeug gemäß einem der Ansprüche 8-14, wobei die Vielzahl von Lücken eine Breite besitzen, die innerhalb des Bereichs zwischen etwa 0,2 mm bis etwa 3 mm ausgewählt ist.

## Revendications

1. Un outil formant râpe chirurgicale (100) pour abraser un os, l'outil comprenant :
un corps généralement allongé (102) ayant une extrémité proximale (104), une extrémité distale (106), et un axe longitudinal, l'extrémité proximale (104) incluant un premier segment proximal (110) ;
une tête (108) formée dans l'extrémité distale du corps (102) ;
une lumière (114) formée à l'intérieur du corps et s'étendant depuis le premier segment proximal (110) jusqu'à la tête ;
la tête incluant en outre :
une ouverture (116) couvrant une partie de la largeur de la tête et en communication avec la lumière ;
une pluralité de dents (118) s'étendent transversalement à l'axe longitudinal et couvrant la largeur de l'ouverture, la pluralité de dents étant espacées longitudinalement par une pluralité d'espaces (120) ;
dans lequel le premier segment proximal du corps (110) est en outre conçu pour communiquer avec une source de vide et dans lequel la lumière est conçue pour transmettre une force de vide appliquée par la source de vide depuis le premier segment proximal (110) jusqu'à la pluralité d'espaces (120).

2. L'outil de la revendication 1, dans lequel le corps comprend en outre un coude positionné de façon proximale par rapport à la tête et dans lequel un axe longitudinal du corps n'est pas aligné avec l'axe longitudinal de la tête.

3. L'outil de la revendication 1, dans lequel le corps inclut en outre un deuxième segment proximal, le deuxième segment proximal et l'extrémité distale du corps s'étendant le long d'un axe longitudinal du corps, le deuxième segment proximal étant par exemple conçu pour se mettre en prise avec une attache pour la manipulation de l'outil, dans lequel par exemple le premier segment proximal est orienté transversalement par rapport à l'axe longitudinal du corps, le premier segment proximal étant par exemple en outre formé avec ou conçu pour recevoir une vis Luer ou tout type d'adaptateur pour s'accoupler avec une vis Luer correspondante ou tout type d'adaptateur en communication avec la source de vide.

4. L'outil de n'importe lesquelles des revendications 1 à 3, dans lequel la lumière est accessible uniquement depuis la pluralité d'ouvertures dans la tête et le premier segment proximal.

5. L'outil de n'importe lesquelles des revendications 1 à 4, dans lequel la lumière est formée d'un seul tenant à l'intérieur du corps.

6. L'outil de n'importe lesquelles des revendications 1 à 5, dans lequel les dents sont conçues pour empêcher une déformation permanente ou une fracture lorsqu'elles sont en contact avec l'os.

7. L'outil de n'importe lesquelles des revendications 1 à 6, dans lequel la pluralité d'espaces possèdent une largeur sélectionnée dans la gamme allant d'environ 0,2 mm à environ 3 mm.

8. Un outil d'abrasion d'os (200), comprenant :
un corps généralement allongé ayant une extrémité proximale (204), une extrémité distale (206), et un axe longitudinal ;
une tête (208) formée au niveau de l'extrémité distale du corps (202) ;
un canal formé à l'intérieur du corps, le canal s'étendant de façon proximale depuis la tête et transversalement à travers une première surface du corps ;
la tête incluant en outre :
une ouverture formée à travers une deuxième surface du corps, opposée à la première surface, et en communication avec le canal, l'ouverture couvrant une partie de la largeur de la tête ;
une pluralité de dents s'étendant transversalement à l'axe longitudinal et couvrant la largeur de l'ouverture, la pluralité de dents étant espacées longitudinalement par une pluralité d'espaces ; et
un tube dimensionné pour être reçu à l'intérieur du canal, le tube s'étendant entre une extrémité proximale et une extrémité distale, l'extrémité distale du tube incluant une ouverture conçue pour communiquer avec l'ouverture dans l'extrémité distale du corps lorsque le tube est inséré à l'intérieur du canal et l'extrémité proximale du tube étant en outre conçue pour communiquer avec une source de vide ;
dans lequel le tube est en outre conçu pour transmettre une force de vide appliquée par la source de vide depuis l'extrémité proximale du tube jusqu'à la pluralité d'espaces lorsque le tube est inséré à l'intérieur du canal.

9. L'outil de la revendication 8, dans lequel le corps comprend en outre un coude positionné de façon proximale par rapport à la tête et dans lequel un axe longitudinal du corps n'est pas aligné avec l'axe longitudinal de la tête.

10. L'outil de la revendication 8, dans lequel l'extrémité proximale du corps et l'extrémité distale du corps s'étendent le long d'un axe longitudinal du corps.

11. L'outil de la revendication 8, dans lequel l'extrémité proximale du corps est conçue pour se mettre en prise avec une attache pour la manipulation de l'outil, dans lequel par exemple le canal augmente en profondeur vers la tête et l'extrémité proximale du tube est orientée dans une direction transversale à l'axe longitudinal du corps lorsque le tube est inséré à l'intérieur du canal.

12. L'outil de n'importe lesquelles des revendications 8 à 11, dans lequel la première extrémité proximale du tube est en outre formée avec ou conçue pour recevoir une vis Luer ou n'importe quel type d'adaptateur pour s'accoupler avec une vis Luer correspondante ou n'importe quel type d'adaptateur en communication avec la source de vide.

13. L'outil de n'importe lesquelles des revendications 8 à 12, dans lequel le tube est substantiellement étanche aux fluides et accessible uniquement depuis l'extrémité proximale du tube et l'extrémité distale du tube.

14. L'outil de n'importe lesquelles des revendications 8 à 13, dans lequel les dents sont dimensionnées de façon à empêcher une déformation ou une fracture lorsqu'elles sont en contact avec l'os.

15. L'outil de n'importe lesquelles des revendications 8 à 14, dans lequel la pluralité d'espaces possèdent une largeur sélectionnée dans la gamme allant d'environ 0,2 mm à environ 3 mm.
